# NEUE EUROPÄISCHE PATENTSCHRIFT

(11) **EP 3 694 349 B2**
(45) Veröffentlichungstag und Bekanntmachung des Hinweises auf die Entscheidung über den Einspruch: **23.10.2024**
(45) Hinweis auf die Patenterteilung: 23.02.2022
(21) Anmeldenummer: 18785944.2
(22) Anmeldetag: 11.10.2018
(51) Int. Cl.: A24F 40/30, A24F 40/50, A61M 15/06, A61M 11/04

(54) **INHALATOR, INSBESONDERE ELEKTRONISCHES ZIGARETTENPRODUKT**
INHALER, PARTICULARLY ELECTRONIC CIGARETTE PRODUCT
INHALATEUR, NOTAMMENT PRODUIT CIGARETTE ÉLECTRONIQUE

(30) Priorität: 13.10.2017 DE 102017123867
(43) Veröffentlichungstag der Anmeldung: 19.08.2020
(73) Patentinhaber: Körber Technologies GmbH, 21033 Hamburg (DE)
(72) Erfinder: KESSLER, Marc, 22415 Hamburg (DE); NIEBUHR, Gunnar, 22605 Hamburg (DE); SCHMIDT, Rene, 21244 Buchholz i.d.N. (DE); WAGENKNECHT, Cameron John, 20249 Hamburg (DE); SCHLÜTER, Björn, 22941 Bargteheide (DE)
(74) Vertreter: Müller Verweyen
(86) Internationale Anmeldenummer: PCT/EP2018/077718
(87) Internationale Veröffentlichungsnummer: WO 2019/072960

(56) Entgegenhaltungen:
- EP-A1- 2 989 912
- EP-A1- 3 170 413
- EP-B1- 2 989 912
- WO-A1-2014/066730
- WO-A1-2015/076515
- WO-A1-2016/090426
- WO-A1-2016/121143
- WO-A1-2017/153270
- WO-A2-2015/038981
- DE-A1- 102016 002 665
- GB-A- 2 524 779
- US-A1- 2006 196 968
- US-A1- 2014 060 554
- US-A1- 2014 366 898
- US-A1- 2016 089 508
- US-A1- 2016 262 454
- US-A1- 2017 251 727
- US-A1- 2017 258 140

## Beschreibung

Die vorliegende Erfindung betrifft einen Inhalator, insbesondere ein elektronisches Zigarettenprodukt, umfassend mindestens eine Verdampfervorrichtung mit mindestens einem elektrischen Verdampfer zum Verdampfen von dem Verdampfer zugeführter Flüssigkeit und eine elektronische Steuervorrichtung zum Steuern und/oder Regeln des Verdampfers.

In aktuellen elektronischen Zigaretten kommen derzeit zwischen einem und bis zu fünf Verdampferköpfe auf Basis des Docht-Wendels-Prinzips zum Einsatz, die ihr Liquid aus einem gemeinsamen Reservoir beziehen. Der Konsument kann lediglich die Gesamtleistung der Verdampfungsvorrichtung und dadurch die erzeugte Dampfmenge einstellen, weitergehende Einstellungen sind nicht möglich.

Die WO 2014/066730 A1 offenbart einen Heizer aus einem Nylonsubstrat und einem Nickelchromdraht.

Die GB 2524779 A offenbart ein Heizelement, das um einen Docht herum angeordnet ist.

In der WO 2015/076515 A1 ist das Heizelement durch ein nicht brennbares Metallnetz gebildet.

Die DE 10 2016 002 665 A1 offenbart ein Heizelement, das in einem Strömungskanal angeordnet ist, so dass das Liquid im Strömungskanal verdampft wird.

WO 2015/038981 A2, US 2016/089508 A1, US 2017/251727 A1, WO 2016/090426 A1, US 2014/366898 A1, US 2017/258140 A1, EP 3 170 413 A1 und EP 2 989 912 A1 offenbaren ein Heizelement in Form einer Heizspule.

Die Aufgabe der Erfindung besteht darin, einen Inhalator mit einer Vielfalt an Einstellmöglichkeiten bereitzustellen, der einen hohen Mehrwert für den Konsumenten aufgrund einer hochgradigen Individualisierbarkeit des Raucherlebnisses in Dampfmenge und Geschmack ermöglicht.

Die Erfindung löst diese Aufgabe mit den Merkmalen des unabhängigen Anspruchs 1. Erfindungsgemäß sind der Verdampfervorrichtung eine Mehrzahl von Flüssigkeiten zugeordnet oder zuordnungsbar, derart, dass die Zusammensetzung und/oder die Dampferzeugungsrate des von dem mindestens einen Verdampfer erzeugten Dampfs/Aerosols gezielt einstellbar und/oder veränderbar ist, indem die Steuervorrichtung zum individuellen Ansteuern des mindestens einen oder des individuell jeweiligen Verdampfers und/oder zum gruppenweisen Ansteuern von Verdampfern eingerichtet ist. Aufgrund der individuellen Ansteuerung des mindestens einen Verdampfers ist es möglich, durch Zuführung gleicher oder unterschiedlicher Flüssigkeiten die Dampfmenge und/oder die Zusammensetzung des von dem mindestens einen Verdampfer erzeugten Dampfs/Aerosols gezielt einzustellen und/oder zu verändern, wodurch das Raucherlebnis individuell durch den Konsumenten eingestellt werden kann.

Die erfindungsgemäße Zuordnung der Mehrzahl von Flüssigkeiten zu dem mindestens einen Verdampfer kann vorteilhaft durch eine Mehrzahl von Flüssigkeitszuführungen zum Zuführen der mehreren Flüssigkeiten zu dem mindestens einen Verdampfer realisiert werden.

Die Erfindung umfasst die Möglichkeit, mit nur einem Verdampfer die Dampfqualität zu beeinflussen, beispielsweise über eine geeignete Frequenzansteuerung des Verdampfers.

Vorzugsweise ist in der elektronischen Steuervorrichtung eine Mehrzahl von an die unterschiedlichen Flüssigkeiten angepassten Parametersätzen gespeichert. Dies ermöglicht es, die Flüssigkeiten bzw. das Flüssigkeitsgemisch jeweils optimal im Hinblick auf die gewünschte Wirkung und/oder das gewünschte Geschmackserlebnis zu verdampfen.

In vorteilhaften Ausführungsformen der Erfindung weist die Verdampfervorrichtung eine Mehrzahl von Verdampfern auf. Dies ermöglicht es, gleiche und/oder unterschiedliche Flüssigkeiten mit einem oder mehreren an die jeweilige Flüssigkeit optimal angepassten Verdampfer zu verdampfen. Zudem ergibt sich aufgrund einer vorteilhaften individuellen Ansteuerbarkeit einzelner Verdampfer mit ggf. unterschiedlichen Heizparametern eine größere Variabilität des Verdampfungsprozesses. Demnach ist vorteilhaft jeder Flüssigkeit mindestens ein eigener Verdampfer zugeordnet.

Eine Mehrzahl von Verdampfern für eine Flüssigkeit kann vorteilhaft eine Vervielfachung bzw. Vergrößerung des Dampfvolumens und/oder zur Leistungssteigerung der Verdampfervorrichtung bewirken. Es kann demnach eine Mehrzahl von Verdampfern zum Verdampfen einer gleichen Flüssigkeit vorgesehen sein, um die Verdampfungsleistung im Hinblick auf eine bestimmte Flüssigkeit zu erhöhen. Es können auch für mehrere oder sämtliche Flüssigkeiten jeweils mehrere Verdampfer vorgesehen sein, um für die entsprechenden Flüssigkeiten jeweils die Verdampfungsleistung zu erhöhen. Die Mehrzahl von Verdampfern ist grundsätzlich unabhängig von der Anbindung an das die Flüssigkeitsreservoire. Die Erhöhung der Anzahl von Verdampfern für eine Flüssigkeit ist vorteilhaft gegenüber einer Vergrößerung des Verdampfers selbst.

Vorteilhaft weist der Inhalator eine Nutzerschnittstelle auf, über welche ein Nutzer, insbesondere der Konsument, die Zusammensetzung des von dem mindestens einen Verdampfer erzeugten Dampfs bzw. Aerosols beeinflussen kann. Die Nutzerschnittstelle ist besonders vorteilhaft eine Drahtlosschnittstelle, beispielsweise eine Bluetooth-Schnittstelle, zur Kommunikation mit einem mobilen Kommunikationsendgerät des Nutzers, insbesondere einem Smartphone. Auf diese Weise kann der Inhalator auf einfach Weise wie gewünscht eingestellt werden.

Eine vorteilhafte Anwendung der Erfindung betrifft ein elektronisches Zigarettenprodukt, kurz eine E-Zigarette. In diesem Fall enthalten die Flüssigkeiten gleiche und/oder unterschiedliche Anteile bzw. Mischungsverhältnisse von 1,2-Propylenglycol, Glycerin, Wasser, mindestens einem Wirkstoff, insbesondere Nikotin, und/oder mindestens einem Aromastoff (Flavour).

Vorzugsweise enthält mindestens eine der Flüssigkeiten mehr als 50% Glycerin, das ein guter Dampfbildner ist.

Weiterhin enthält mindestens eine der Flüssigkeiten mindestens 1% Nikotin und mindestens eine der Flüssigkeiten kein Nikotin. Dies ermöglicht eine hohe Variabilität des Nikotingehalts im Dampf / Aerosol.

Prozentangaben für Flüssigkeitsanteile können im Rahmen dieser Anmeldung generell Volumen- und/oder Gewichtsprozente sein.

In einer Ausführungsform ist jede der dem mindestens einem Verdampfer zugeführten Flüssigkeiten frei von Nikotin, um die Erzeugung eines nikotinfreien Dampfes/Aerosols zu ermöglichen und/oder eine übermäßige Erhitzung von Nikotin zu vermeiden.

Des Weiteren enthält mindestens eine der Flüssigkeiten ein Flavour und mindestens eine der Flüssigkeiten kein Flavour, oder ein anderes Flavour. Dies ermöglicht eine hohe Variabilität eines Gehalts an einem oder mehreren Flavours (Geschmacksstoff) im Dampf bzw. Aerosol.

Vorteilhaft weist der Inhalator eine Mehrzahl von Flüssigkeitsspeichern oder Reservoirs zum Speichern der mehreren Flüssigkeiten auf. Durch den Einsatz eines oder mehrerer Flüssigkeitsspeicher, welche vorteilhaft einem oder mehreren Verdampfern und/oder einem oder mehreren Verdampfer-Trägern individuell zuordnungsbar sind, können einzelne Verdampfer und/oder Verdampfer-Träger gleiche Liquids und/oder unterschiedliche Liquids mit unterschiedlicher Zusammensetzung verdampfen.

Vorteilhaft enthält mindestens eine der Flüssigkeiten mehrheitlich eine oder mehrere Komponenten aus der Gruppe 1,2-Propylenglycol und eine andere der Flüssigkeiten enthält mehrheitlich Glycerin. In mehreren Reservoirs können dann Flüssigkeiten mit unterschiedlichen Mischungsverhältnissen bereitgestellt werden, so dass in einer bei der Verdampfung erzeugten Dampfmischung das Verhältnis der Aroma- und/oder Wirkstoffe einstellbar und/oder veränderbar ist. Vorteilhaft kann das Verhältnis der Aroma- und/oder Wirkstoffe der Dampfmischung kontinuierlich verstellt werden.

Dazu können in den beiden Flüssigkeiten voneinander unterschiedliche Mischungsverhältnisse von einer oder mehrerer Komponenten aus der Gruppe 1,2-Propylenglycol und Glycerin vorliegen. In einer Ausführungsform ist das Verhältnis der Anteile von einer oder mehrerer Komponenten aus der Gruppe 1,2-Propylenglycol und Glycerin in der einen Flüssigkeit größer als 50/50 und in der anderen Flüssigkeit kleiner als 50/50, vorteilhaft ist das Verhältnis der Anteile in der einen Flüssigkeit größer als 75/25 und in der anderen Flüssigkeit kleiner als 25/75, beispielsweise ist das Verhältnis der Anteile in der einen Flüssigkeit 90/10 und in der anderen Flüssigkeit 10/90. Das Verhältnis der Anteile kann sich auf das Verhältnis der Massen-, Stoffmengen- und/oder Volumenanteile beziehen.

In einer Ausführungsform weist der Inhalator wenigstens eine Kapsel zum Speichern eines aroma- und/oder wirkstoffhaltigen, insbesondere nikotinhaltigen, Feststoffes auf. Dabei ist die Kapsel vorzugsweise von dem von dem mindestens einen Verdampfer erzeugten Dampf/Aerosol durchströmbar, um eine Überhitzung des Nikotins zu vermeiden. In dieser Ausführungsform ist die Kapsel vorzugsweise stromabwärts des Verdampfers angeordnet beziehungsweise nachgelagert. Der von dem Verdampfer erzeugte Dampf beziehungsweise das Aerosol durchströmt die Kapsel, wobei das Nikotin durch den Dampf/das Aerosol aus dem nikotinhaltigen Feststoff herausgelöst und zum Mundstück transportiert wird. Insbesondere wird das nikotinfreie Liquid verdampft und strömt durch die Kapsel mit aufbereitetem Tabak und/oder Nikotin zur Aufbereitung des Dampfes/Aerosols hindurch, bevor es vom Konsumenten konsumiert wird. Dabei kann für die Geschmacksentwicklung das Verhältnis zwischen einer oder mehrerer Komponenten aus der Gruppe 1,2-Propylenglycol und Glycerin des Dampfes/Aerosols von Bedeutung sein.

Der Feststoff kann ein tabakhaltiges Material sein, beispielsweise ein nikotinhaltiges Granulat und/oder Kügelchen. In dieser Ausführungsform ist vorteilhaft in jeder dem Verdampfer zugeführten Flüssigkeiten kein Nikotin vorhanden. In dieser Ausführungsform ist der Inhalator ein Hybrid-System, welches dazu eingerichtet ist, eine Flüssigkeit zu verdampfen und Nikotin aus einem Feststoff zu lösen.

Die Erfindung stellt des Weiteren vorzugsweise ein Computerprogrammprodukt, insbesondere eine Anwendungssoftware oder App, für ein mobiles Kommunikationsendgerät, insbesondere ein Smartphone bereit. Das Computerprogrammprodukt ist zum Steuern und/oder Einstellen des Inhalators insbesondere über eine Drahtlosschnittstelle, beispielsweise eine Bluetooth-Schnittstelle, eingerichtet. Kommunikationsendgeräte wie Smartphones verfügen über eine solche Drahtlosschnittstelle und erlauben über eine grafische Bedieneroberfläche auf einem in der Regel berührungsempfindlichen Bildschirm eine intuitive und detaillierte Steuerung und/oder Einstellung des Inhalators, sowie auch die Anzeige von den Inhalator betreffender Information.

Vorzugsweise weist das Computerprogrammprodukt Auswahlelemente zum Auswählen eines aus einer Mehrzahl von voreingestellten Verdampfungsprofilen, und entsprechende Ansteuerung des Inhalators, auf. Beispielsweise kann ein Verdampfungsprofil mit wenig Wirkstoff und /oder geringem Throat Hit, ein Verdampfungsprofil mit viel Wirkstoff und /oder hohem Throat Hit, und ggf. ein dazwischen liegendes mittleres Verdampfungsprofil vorgesehen sein. Selbstverständlich können mehr als drei unterschiedliche Verdampfungsprofile vorgesehen sein. Im Allgemeinen können voreingestellte Verdampfungsprofile zum Einstellen von mindestens zwei, vorzugsweise mindestens drei unterschiedlichen Stärkegraden an Wirkstoffgehalt und/oder Throat Hit vorgesehen sein.

Eine relativ geringe Zahl an voreingestellten Verdampfungsprofilen werden von Konsumenten bevorzugt, die schnell und unkompliziert einige unterschiedliche Raucherlebnisse einstellen wollen und keine hochgradig differenzierten Einstellmöglichkeiten benötigen.

Vorzugsweise umfasst das Computerprogrammprodukt, insbesondere die Anwendungssoftware bzw. die graphische Benutzeroberfläche derselben, Einstellelemente zum Einstellen individueller Verdampfungsparameter des Inhalators. Dieser manuelle Modus richtet sich an Konsumenten, die ein hohes Maß an differenzierten Einstellmöglichkeiten wünschen.

In diesem Modus umfasst das Computerprogrammprodukt, insbesondere die Anwendungssoftware bzw. die graphische Benutzeroberfläche derselben, vorteilhaft Einstellelemente zum Einstellen des Anteils einzelner Flüssigkeiten an dem erzeugten Dampf/Aerosol; Einstellelement zum Einstellen eines Throat Hit; und/oder ein Einstellelement zum Einstellen der insgesamt erzeugten Dampfmenge.

Vorteilhaft umfasst das Computerprogrammprodukt, insbesondere die Anwendungssoftware bzw. die graphische Benutzeroberfläche derselben, ein Einstellelement zum Einstellen des Aroma- und/oder Wirkstoffgehaltes, um den Konsumenten eine einfache Einstellmöglichkeit des Aroma- und/oder Wirkstoffgehaltes zu ermöglichen. Beispielsweise kann durch das Einstellelement das Mischungsverhältnis von einer Dampfmischung aus einer oder mehrerer Komponenten aus der Gruppe 1,2-Propylenglycol und Glycerin einstellbar sein.

Ein höherer Anteil an Glycerin führt zu mehr Dampfentwicklung. Ein höherer Anteil an einer oder mehrerer Komponenten aus der Gruppe 1,2-Propylenglycol führt zu einer stärkeren Wirkung im Rachen beziehungsweise Throat Hit. Das Mischungsverhältnis der Dampfmischung kann insbesondere durch den Konsumenten individuell auf eine dem Konsumenten angenehme Mischung während des Konsumierens der Kartusche einstellbar und/oder veränderbar sein.

In einer Ausführungsform kann der Dampfmischung Nikotin zugegeben werden. Die Zugabe von Nikotin kann durch ein beziehungsweise das Einstellelement möglich sein. Vorteilhaft kann der Sättigungsgrad der Dampfmischung mit Nikotin durch das Mischungsverhältnis veränderbar und/oder einstellbar sein. Damit kann das Verdunstungsverhalten der Aroma- und/oder Wirkstoffe, insbesondere Nikotin, zeitlich angepasst werden und somit der zeitliche Verlauf von individuellen Geschmackserlebnis und Wirkstoffgabe beeinflusst werden.

In einer Ausführungsform liegt das Nikotin in einer nikotinhaltige Feststoffe speichernden Kapsel vor. Das Einstellelement kann die Durchströmung der Kapsel, insbesondere die Geschwindigkeit und Temperatur eines durchströmenden Luftstroms, und somit das Lösen von Nikotin und die Zugabe von Nikotin in den Luftstrom beziehungsweise den Dampf/das Aerosol beeinflussen.

Die Auswahlwählbarkeit und/oder Einstellbarkeit der Verdampfungsparameter ist dabei untereinander gekoppelt. Das Computerprogrammprodukt berücksichtigt sämtliche Abhängigkeiten von Verdampfungsparametern untereinander, und passt die visuelle Darstellung in der grafischen Benutzeroberfläche entsprechend an.

Vorteilhaft ist zur Information des Konsumenten auch eine Anzeige des Ladezustands des Energiespeichers in dem Inhalator und/oder eine Anzeige der restlichen Liquidmenge in dem oder den Reservoirs des Inhalators, und/oder eine Anzeige der verbleibenden Züge oder Puffs durch den Konsumenten vorgesehen. Die Anzahl der verbleibenden Puffs kann dabei abhängig von den gewählten Einstellungen, dem Rauchverhalten, beispielsweise der durchschnittlichen individuellen Zuglängen, und dem Energiebedarf berechnet werden.

Vorzugsweise kann das Computerprogrammprodukt ein Schaltelement zum Einstellen des Zugwiderstands des Inhalators aufweisen, was dem Konsumenten eine weitere Variabilität des Raucherlebnisses verschafft. Beispielsweise kann mit dem Schaltelement ein einer klassischen Zigarette entsprechender Zugwiderstand und/oder ein möglichst geringer Zugwiderstand einstellbar sein.

Die Erfindung wird im Folgenden anhand bevorzugter Ausführungsformen unter Bezugnahme auf die beigefügten Figuren erläutert.

Dabei zeigt
- Fig. 1: eine schematische Darstellung einer E-Zigarette in einer Ausführungsform der Erfindung;
- Fig. 2: eine schematische Darstellung einer E-Zigarette in einer weiteren Ausführungsform der Erfindung;
- Fig. 3-10: schematische Darstellungen unterschiedlicher Verdampferkonzepte für eine E-Zigarette;
- Fig. 11, 12: Bildschirmdarstellungen einer Smartphone-App zum Bedienen und Einstellen einer E-Zigarette; und
- Fig. 13: eine schematische Querschnittsansicht einer beispielhaften Verdampfereinheit für eine E-Zigarette.

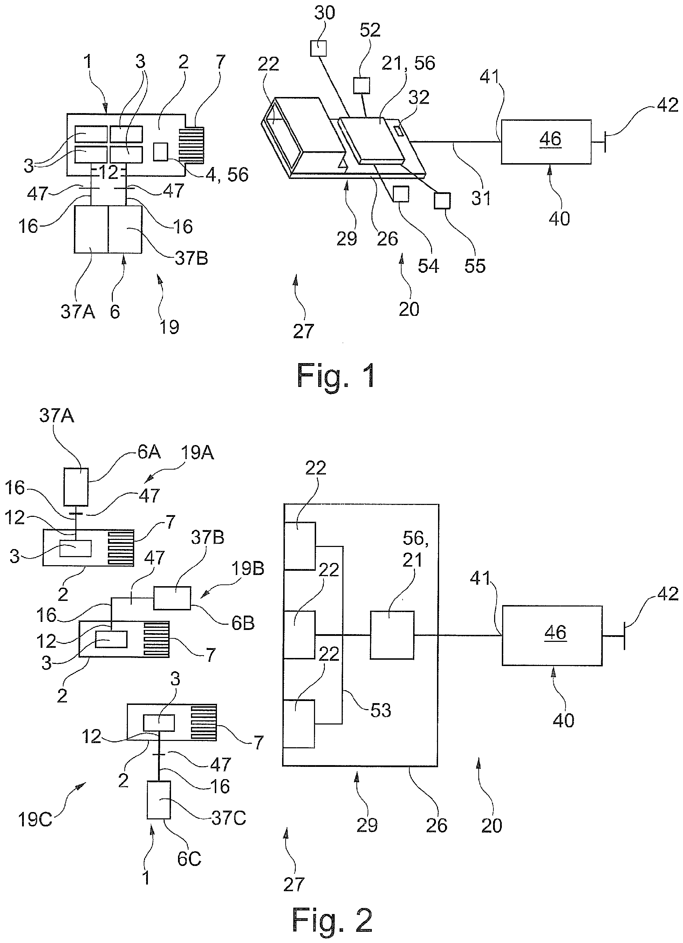

Das in Figur 1 beispielhaft gezeigte Komponentensystem für einen Inhalator 27 umfasst eine Verdampfervorrichtung 1 und ein vorteilhaft wiederverwendbares Basisteil 20.

Die Verdampfervorrichtung 1 umfasst mindestens einen Träger 2, der auch als Trägerplatte bezeichnet werden kann, und mindestens einen mit dem Träger 2 zu verbindenden oder verbundenen Flüssigkeitsspeicher 6. Jeder Träger 2 weist mindestens einen Verdampfer 3 auf, der zum Verdampfen von dem Verdampfer 3 aus dem mindestens einen Flüssigkeitsspeicher 6 zugeführter Flüssigkeit dient. Das dem Verdampfer 3 zugeführte Liquid wird somit durch den Verdampfer 3 in Dampf/Aerosol umgesetzt. Der insbesondere elektrische Verdampfer 3 weist mindestens ein, vorzugsweise eine Mehrzahl von elektrischen Widerstands-Heizelementen auf. Der Verdampfer 3 ist vorzugsweise in Bezug zu einer Oberseite des Trägers 2 angeordnet, so dass von dem Verdampfer 3 erzeugter Dampf und/oder Aerosol aus der Oberseite des Trägers 2 austritt.

Die Verdampfervorrichtung 1 ist vorteilhaft aus einer oder mehreren baulichen Einheiten bzw. Verdampfereinheiten 19 gebildet, bestehend aus jeweils mindestens einem Träger 2 mit jeweils mindestens einem Verdampfer 3 und jeweils mindestens einem Flüssigkeitsspeicher 6. Die Verdampfereinheiten 19 sind vorteilhaft auswechselbare Kartuschen.

Auf dem Träger 2 ist vorteilhaft eine digitalelektronische Steuereinrichtung 4, beispielsweise eine anwendungsspezifische integrierte Schaltung (ASIC), angeordnet. Die Verdampfer 3 können durch die elektronische Steuereinrichtung 4 individuell oder in Gruppen angesteuert und mit Strom aus einem Energiespeicher 46 beheizt werden, um an den Heizelementen anliegende Flüssigkeit zu verdampfen.

Der Träger 2 weist ein elektrisches Steckverbinderteil 7, hier in Form eines elektrischen Steckers, mit einer Mehrzahl von elektrischen Kontakten 10 auf. Die Kontakte 10 sind mittels elektrischer Leitungen mit der elektronische Steuereinrichtung 4 verbunden, um Sensorsignale, Steuersignale und/oder elektrische Energie zwischen dem Basisteil 20 und der Verdampfereinheit 19 zu übertragen. Der vorteilhaft durchgängige, formstabile Träger 2 kann aus einem geeigneten, vorteilhaft nichtleitenden Material bestehen, beispielsweise Keramik oder einem geeigneten Kunststoff, insbesondere PEEK, oder faserverstärktem Kunststoff, beispielsweise PCB-Material. An der Verdampfervorrichtung 1 können Sensoren, beispielsweise Temperatursensor zum Messen der Heiztemperatur und/oder ein Drucksensor zum Messen des Strömungsdrucks vorgesehen sein.

Das Basisteil 20 umfasst eine Steuereinheit 29 und eine mit der Steuereinheit 29 verbundene oder zu verbindende Energiespeichereinheit 40. Die Steuereinheit 29 umfasst eine elektronische Steuerung 21 und eine mit der elektronischen Steuerung 21 elektrisch verbundene elektrische Steckbuchse 22. Die elektronische Steuerung 21 und die elektrische Steckbuchse 22 sind vorteilhaft auf einer gemeinsamen Leiterplatte 26 angeordnet. Die Gesamtheit aus elektronischer Steuereinrichtung 4 und elektronischer Steuerung 21 wird im Rahmen dieser Anmeldung als elektronische Steuervorrichtung 56 des Inhalators 27 bezeichnet.

Der elektrische Stecker 7 und die elektrische Steckbuchse 22 sind einander entsprechend eingerichtet, so dass durch Einstecken des Steckers 7 in die Steckbuchse 22 eine elektrische Verbindung zwischen der Verdampfervorrichtung 1 und dem Basisteil 20 zur Übertragung von Signalen, Daten und/oder elektrischer Leistung hergestellt wird. In der Verdampfervorrichtung 1 werden die Versorgungsströme und Signale von dem Stecker 7 an den mindestens einen Verdampfer 3 und/oder an Sensoren weitergeleitet. Vorteilhaft weisen der Stecker 7 und die Steckbuchse 22 jeweils die gleiche Anzahl von elektrischen Kontakten 10 auf. Zum Verbinden der Verdampfervorrichtung 1 mit dem Basisteil 20 wird die Verdampfervorrichtung 1 parallel zur Längsachse des Basisteils 20 in dieses eingeschoben, ggf. auch über einen Adapter bzw. ein Verlängerungsteil, wodurch der Stecker 7 in die Steckbuche 22 eingeschoben und die elektrische Verbindung hergestellt wird.

In der elektronischen Steuereinrichtung 4 der Verdampfervorrichtung 1 ist vorteilhaft eine Kennung bzw. ID (Identifizierungsinformation) der Verdampfervorrichtung 1 dauerhaft gespeichert. Infolge des Verbindens einer Verdampfervorrichtung 1 mit einem Basisteil 20 durch Einstecken des Steckers 5 in die Steckbuchse 22 kann die elektronische Steuerung 21 die Kennung aus der Steuereinrichtung 4 auslesen und eine typgenaue individuelle Steuerung des jeweiligen Verdampfers 3 auf der Verdampfervorrichtung 1 durchführen oder veranlassen, etwa durch Übermittlung von Steuer- und/oder Regelbefehlen an die Steuereinrichtung 4.

In der elektronischen Steuerung 21 des Basisteils 20 sind vorzugsweise Steuerdaten für eine Mehrzahl von Kennungen entsprechend einer Mehrzahl von unterschiedlichen Verdampfern bzw. Verdampfertypen und/oder Flüssigkeiten gespeichert, beispielsweise in Form einer Datenbank. Wenn die elektronische Steuerung 21 eine Kennung aus dem Speicher 14 der Verdampfervorrichtung 1 ausliest, kann sie die dieser Kennung zugeordneten Steuerdaten aus der Datenbank abrufen und die Steuerung des Verdampfers 3 typgerecht und angepasst an die jeweils zu verdampfende Flüssigkeit durchführen.

Die Energiespeichereinheit 40 umfasst einen Energiespeicher 46, eine Batterieschnittstelle 41 zum Verbinden der Steuereinheit 29 mit der Energiespeichereinheit 40 über elektrische Leitungen 31, und eine Ladeschnittstelle 42. Die Steuereinheit 29 wird über die Batterieschnittstelle 41 mit Strom versorgt. Des Weiteren können über die Batterieschnittstelle 41 analoge und/oder digitale Signale zwischen der Energiespeichereinheit 40 und der Steuereinheit 29 übertragen werden. In einer vorteilhaften Ausführungsform umfassen die elektrischen Leitungen 31 einen digitalen Datenbus. Über die elektrische Verbindung 31 zwischen Basisteil 20 und Energiespeichereinheit 40 lassen sich beispielsweise Informationen über den Ladezustand des Energiespeichers 46 oder Diagnosedaten zwischen der Steuereinheit 29 und der Energiespeichereinheit 40 übermitteln.

Der Energiespeicher 46 kann eine Einwegbatterie oder ein wiederaufladbarer Akku sein, beispielsweise ein Lithium-Ionen Akku. In dem gezeigten Ausführungsbeispiel ist der Energiespeicher 46 ein wiederaufladbarer Akku, welcher sich über die Ladeschnittstelle 42, beispielsweise eine USB-Schnittstelle, laden lässt.

Jede Verdampfereinheit 19 weist vorteilhaft eine standardisierte Flüssigkeitsschnittstelle 47 zur Anbindung des mindestens einen Flüssigkeitsspeichers oder -tanks 6 an den Träger 2 auf. Die Flüssigkeitsschnittstelle 47 ist vorteilhaft an der Unterseite, bzw. auf der dem Verdampfer 3 entgegengesetzten Seite des Trägers 2 angeordnet. An der Flüssigkeitsschnittstelle 47 wird demnach das Liquid aus dem oder den Reservoirs 37A, 37B bereitgestellt und durch eine vorteilhafte Durchgangsöffnung durch den Träger 2 zu dem oder den Verdampfern geleitet. Die Flüssigkeitsschnittstelle 47 kann beispielsweise mittels eines Dichtelements abgedichtet sein.

Die Verbindung zwischen dem mindestens einen Flüssigkeitsspeicher 6 und dem Träger 2 ist vorteilhaft reversibel, um den Anwender das Auswechseln des oder der Flüssigkeitsspeicher 6 zu ermöglichen. Der mindestens eine Flüssigkeitsspeicher 6 kann wiederauffüllbar (Mehrwegteil) oder als Einwegteil ausgeführt sein. Träger 2 und Flüssigkeitsspeicher 6 können eine für den Konsumenten unlösbare Einheit bilden, die als Einwegteil oder Mehrwegteil ausgeführt sein kann.

Jeder Flüssigkeitsspeicher 6 ist über eine zugeordnete Flüssigkeitszuführung 16 mit einem oder mehreren Verdampfern 3 verbunden, um Flüssigkeit von einer Öffnung des Flüssigkeitstanks 6 zu dem oder den Verdampfern 3 zu transportieren und dort zu verdampfen. Unter jedem Verdampfer 3, oder zwischen jedem Verdampfer 3 und dem Flüssigkeitsspeicher 6, d.h. in der Flüssigkeitszuführung 16 und beispielsweise in einer Durchgangsöffnung des Trägers 2, ist vorteilhaft ein Kapillarelement 12 vorgesehen, das Flüssigkeit mittels Kapillarwirkung, beispielsweise mithilfe von Mikrokanälen, von dem Flüssigkeitsspeicher 6 zu dem Verdampfer 3 fördert, um die Benetzung des Verdampfers 3 und die kontinuierliche Nachförderung von Liquid sicherzustellen. Das Kapillarelement 12 kann beispielsweise ein Porenelement mit optimierter Porengröße, ein offenporiges geschäumtes Element, ein Schwammelement und/oder eine Lamellenstruktur umfassen.

Auf der der Flüssigkeitsschnittstelle 47 gegenüberliegenden Seite des Trägers 2 wird über einen Luftstrom das verdampfte Liquid in Form von Dampf und/oder Aerosol abgeführt, um von dem Konsumenten inhaliert zu werden.

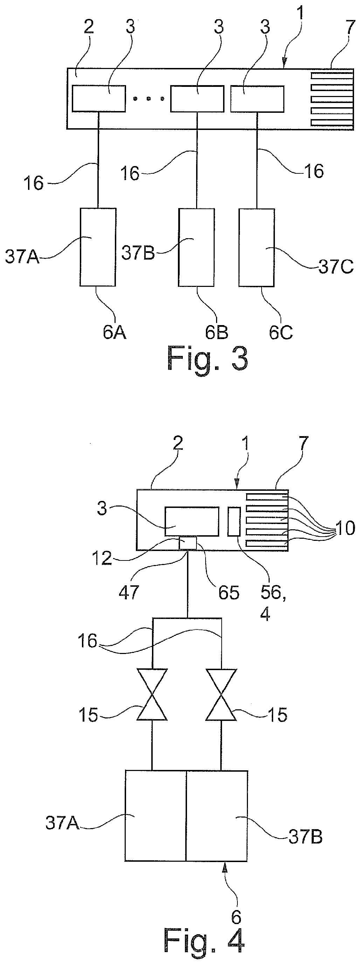

Das vorliegende Konzept sieht demnach den Einsatz eines oder mehrerer Verdampfer 3 auf einem oder mehreren, vorteilhaft keramischen oder aus einem temperaturbeständigen Polymer wie beispielsweise PEEK bestehenden Trägern 2 vor. Jeder Träger 2 verfügt über eine standardisierte Schnittstelle 7 zur elektrischen Kontaktierung des oder der Verdampfer 3. Auf jeweils einem Träger 2 können sowohl ein Verdampfer 3 (siehe Figuren 2 und 4 bis 7) als auch mehrere Verdampfer 3 (siehe Figuren 1 und 3) integriert sein.

In der Ausführungsform nach Figur 1 sind die Verdampfer 3 beispielsweise in Matrixform, hier beispielsweise vier Verdampfer 3 in 2x2 Matrixform, angeordnet.

Das Komponentensystem für eine E-Zigarette 27 in Figur 2 zeigt die Möglichkeit zur Integration von beispielsweise drei Kartuschen 19 mit jeweils einem Träger 2 und beispielsweise jeweils einem Verdampfer 3 auf jedem Träger 2. Jeder Träger 2 enthält somit einen Verdampfer 3 mit jeweils einem eigenen Tank 6A, 6B, 6C. So lassen sich mit diesem System drei verschiedene Liquids A, B, C zu unterschiedlichen, vom Konsumenten individuell einstellbaren Anteilen, und/oder nacheinander geschaltet, in Dampf freisetzen.

In dieser Ausführungsform und vorteilhaft im Allgemeinen weist das Basisteil 20 bzw. die Steuereinheit 29 eine der Anzahl der Kartuschen 19 entsprechende Zahl von Steckverbinderteilen 22, hier Steckbuchsen, auf, um den individuellen Austausch einzelner Kartuschen 19 zu ermöglichen. Die Steckbuchsen 22 sind über einen Verteiler 53 mit der elektronischen Steuerung 21 des Basisteils 20 verbunden.

In der Ausführungsform nach Figur 3 sind die Verdampfer 3 beispielsweise in einer Reihe angeordnet. Andere Anordnungen einer Mehrzahl von Verdampfern 3 auf einem Träger 2 sind möglich.

In den Ausführungsformen gemäß Figuren 1 bis 3 ist jedem Reservoir 37A, 37B, ... mindestens ein Verdampfer 3 zugeordnet, der ausschließlich von dem zugeordneten Reservoir 37A, 37B, ... mit Liquid versorgt wird. Dies hat den Vorteil, dass auf die jeweilige Flüssigkeit A, B, C optimierte Verdampfer 3 verwendet werden können. In der Ausführungsform gemäß Figur 1 sind jedem Reservoir 37A, 37B, ... beispielsweise zwei Verdampfer 3 zugeordnet. Das Dampfvolumen bzw. die Verdampfungsleistung kann für jedes Reservoir 37A, 37B, ... durch Erhöhung der Zahl der Verdampfer 3, hier beispielsweise durch mehr als zwei Verdampfer 3 pro Reservoir 37A, 37B, ... gesteigert werden.

Es ist aber nicht ausgeschlossen, dass derselbe Verdampfer 3 mit Liquid aus einer Mehrzahl von Reservoirs 37A, 37B, ... versorgt wird, so dass der Mehrzahl von Reservoirs 37A, 37B, ... der gleiche Verdampfer 3 zugeordnet ist. Eine entsprechende Ausführungsform ist beispielhaft in Figur 4 für zwei Reservoirs 37A, 37B gezeigt. In diesem Fall sind in den Zuführungen 16 der Mehrzahl von Reservoirs 37A, 37B vorteilhaft steuerbare Ventile 15, insbesondere Dosierventile, oder sonstige Elemente zum individuellen Steuern / Regeln des Durchflusses durch die Zuführungen 16 vorgesehen, um die Dampfzusammensetzung des Verdampfers 3 entsprechend einstellen und verändern zu können.

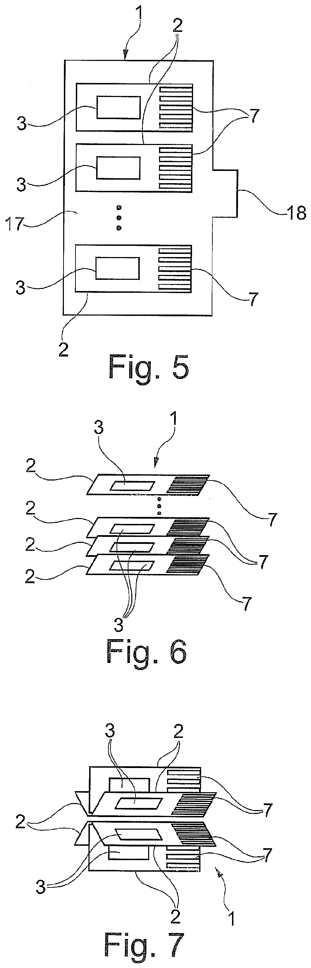

In weiteren Ausführungsformen kann die Verdampfervorrichtung 1 eine Mehrzahl von Trägern 2 mit jeweils einem oder mehreren Verdampfern 3 aufweisen, siehe Figuren 5 bis 7. Die Mehrzahl von Trägern 2 kann vorteilhaft zu einer größeren Verdampferbaugruppe (Komplex) kombiniert werden. In dem Ausführungsbeispiel der Figur 5 ist eine Mehrzahl von Trägern 2 auf einem Hauptträger 17 angeordnet, hier beispielsweise in einer Reihe, und mit diesem elektrisch und mechanisch verbunden. Der Hauptträger 17 kann vorteilhaft ein Steckverbinderteil 18 zum Zusammenwirken mit dem Steckverbinderteil 22 des Basisteils 20 aufweisen. Das Steckverbinderteil 18 ist vorteilhaft mit den Steckverbinderteilen 7 der einzelnen Träger 2 elektrisch verbunden. Auf dem Hauptträger 17 können nicht gezeigte Steckverbinderteile zum Zusammenwirken mit den Steckverbinderteilen 7 der der einzelnen Träger 2 vorgesehen sein.

In dem Ausführungsbeispiel der Figur 6 ist eine Mehrzahl von Trägern 2 in der Form eines Stapels, d.h. parallel übereinander und beabstandet zueinander, angeordnet.

In dem Ausführungsbeispiel der Figur 7 ist eine Mehrzahl von beispielsweise sechs Trägern 2 sternförmig mit gleichen Winkelabständen zueinander angeordnet, was bei zylindrischer Geometrie oder Stabgeometrie der Verdampfervorrichtung 1 oder des Inhalators 27 vorteilhaft sein kann.

Die in den Figuren 1 bis 7 gezeigten Geometrien zur Anordnung der Verdampfer 3 und der Träger 2 sind beispielhaft zu verstehen; die Umsetzung der Erfindung ist nicht auf die dargestellten Beispiele beschränkt.

Der oder die Verdampfer 3 auf dem oder den Trägern 2 sind als Komplex, einzeln oder abschnittsweise, d.h. in beliebiger Auswahl, ansteuerbar. Die Träger 2 sind aufgrund ihrer standardisierten Schnittstelle 7 innerhalb eines Komplexes jeweils individuell austauschbar. Jeder Träger 2 innerhalb eines Komplexes, und/oder jeder Verdampfer 3 eines Trägers 2, ist individuell elektrisch ansteuerbar, was eine differenzierte Steuerung der Verdampfung ermöglicht.

Die Verdampfungsvorrichtung 1 weist insgesamt eine Mehrzahl von Flüssigkeitsreservoirs 37A, 37B, ... auf, die unterschiedlich zusammengesetzte Flüssigkeiten enthalten, und eine Mehrzahl von entsprechenden Flüssigkeitszuführungen 16 von den Reservoirs 37A, 37B, ... zu dem oder den Verdampfern 3. Die Flüssigkeiten in den Reservoirs 37A, 37B, ... umfassen vorteilhaft eine oder mehrere Komponenten, wobei die Komponenten 1,2-Proplyenglykol, Glycerin, Wasser, mindestens einen Wirkstoff, insbesondere Nikotin, und/oder mindestens einen Aromastoff (Flavour) in unterschiedlichen Mischungsverhältnissen umfassen.

Die Reservoirs 37A, 37B, ... sind in einem oder mehreren Flüssigkeitsspeichern 6 ausgebildet. Insbesondere kann ein Mehrkammertank 6 zur Ausbildung mehrerer Reservoirs 37A, 37B, ... vorgesehen sein. Beispielhaft ist in den Figuren 1 und 4 jeweils ein Zweikammertank 6 zur Ausbildung zweier Reservoirs 37A, 37B vorgesehen. Selbstverständlich kann ein Flüssigkeitstank 6 auch mehr als zwei Reservoirs 37A, 37B umfassen.

Das Komponentensystem für die E-Zigarette 27 in Figur 1 zeigt eine Verdampferkartusche 19 mit vier Verdampfern 3 auf einem Träger 2. Der Träger 2 ist an einen Tank 6 mit zwei abgetrennten Kammern gekoppelt, so dass jeweils zwei Verdampfer 3 einem Reservoir 37A bzw. 37B zugeordnet sind.

In den Ausführungsformen gemäß Figuren 2 und 3 sind die Reservoirs 37A, 37B, 37C mittels Einkammertanks 6A, 6B, 6C realisiert. D.h. jeder Tank 6A, 6B, 6C weist nur eine Kammer zur Ausbildung eines Reservoirs 37A, 37B, 37C auf.

Mischformen zwischen Ein- und Mehrkammertanks sind möglich, beispielsweise könnte in Figur 2 oder Figur 3 ein Zweikammertank und ein Einkammertank vorgesehen sein.

Durch den Einsatz mehrerer Reservoirs 37A, 37B, ... und einer Mehrzahl entsprechender Flüssigkeitszuführungen 16 können der oder die Verdampfer 3 unterschiedliche in den Reservoirs 37A, 37B, ... enthaltene Liquids mit unterschiedlicher Zusammensetzung verdampfen. Auf diese Weise kann die Zusammensetzung des von dem oder den Verdampfern 3 erzeugten Dampfs bzw. Aerosols gezielt eingestellt werden. Des Weiteren kann durch gezielte Verdampfung unterschiedlicher Flüssigkeiten die Dampf-/Aerosolzusammensetzung gezielt verändert werden.

Es können auch wahlweise mehrere Liquids gleicher Zusammensetzung in dem erfindungsgemäßen Inhalator bereitgestellt werden, beispielsweise zwei Liquids mit gleicher Zusammensetzung und ein Liquid mit anderer Zusammensetzung. Daraus resultiert ebenfalls eine individuelle Zusammensetzbarkeit des Dampfes, über die der Benutzer entscheiden kann, der Flüssigkeitsreservoirs 37A, 37B... in seinen Inhalator, insbesondere ein elektronisches Zigarettenprodukt, einsetzt. Bei einem Inhalator für medizinische Anwendungen kann dies zum Beispiel nach Vorschrift eines Arztes geschehen.

Die Ansteuerung des oder der Verdampfer 3, und damit die Verdampfung, kann vorteilhaft mit unterschiedlichen, zumindest teilweise an das jeweilige Liquid angepassten Parametern geschehen.

In einer beispielhaften Umsetzung können jeweils ein bis vier Verdampfer 3 auf einem Träger 2 integriert sein. Jeder Verdampfer 3 kann über eine individuelle Ansteuerung und eine individuelle Liquidversorgung 16 verfügen. So ist es möglich, bis zu vier unterschiedliche Liquids pro Inhalator 27 zum Einsatz zu bringen.

In der Ausführungsform gemäß Figur 1 sind beispielsweise vier Verdampfer 3 auf einem Träger 2 integriert. Dabei sind hier beispielhaft jeweils zwei Verdampfer 3 einem Reservoir 37A, 37B zugeordnet.

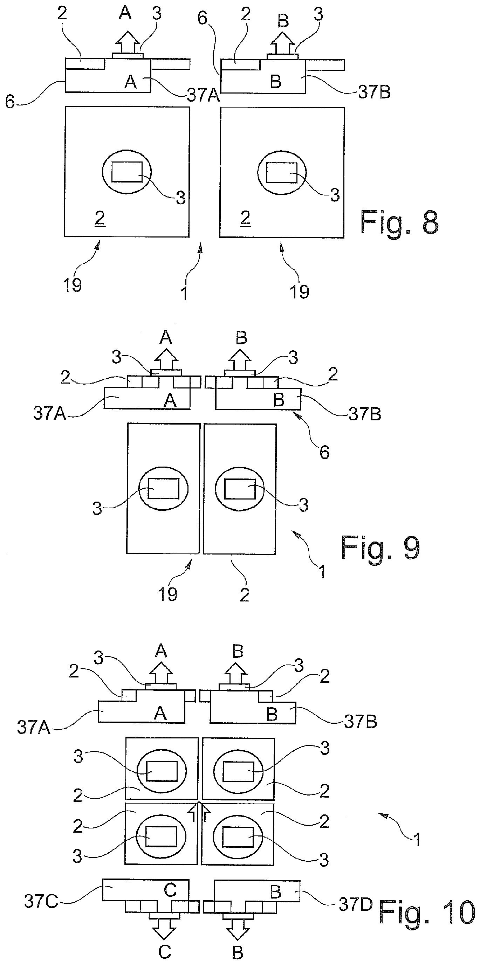

Im Folgenden wird eine beispielhafte Umsetzung der Erfindung anhand der Figuren 8 bis 10 erläutert. Dargestellt sind zwei Liquids A, B (Figuren 8, 9) oder drei Liquids A, B, C (Figur 10), dies gilt aber nicht als Einschränkung.

Beispielhaft kann Liquid A geschmacklich neutral ohne Flavour sein und zu einem erheblichen oder überwiegenden Anteil, beispielsweise zu mindestens 50%, vorzugsweise zu mindestens 60%, insbesondere zu mindestens 70% aus Glycerin bestehen. Liquid B kann geschmacklich neutral oder mit einem Flavour B ausgestattet sein und gleichzeitig Nikotin enthalten. Liquid C enthält vorteilhaft kein Nikotin, aber einen Flavour C, welcher sich gegebenenfalls von dem Flavour B unterscheidet.

Die einzelnen Liquids als Bestandteile des entstehenden Dampfes haben folgende Aufgaben. Aufgabe des Anteils von Liquid A am Dampf ist es, für eine einstellbare schlagartige Wirkung in der Kehle des Konsumenten (sogenannter Throat Hit) und/oder für eine zusätzliche Dampfmenge pro Zug zu sorgen. Aufgabe des Anteils von Liquid B am Dampf ist es, für eine einstellbare Nikotinmenge pro Zug zu sorgen. Aufgabe des Anteils Liquid C und ggf. von Liquid B am Dampf ist es, für eine einstellbare Geschmacksentfaltung pro Zug zu sorgen. Durch die individuelle Ansteuerbarkeit der Verdampfer 3 durch den Konsumenten kann flexibel die entstehende Dampfmenge, Wirkstoffmenge und/oder Aromakombination eingestellt werden, d.h. der Nutzer kann bestimmen, welchen Anteil des entstehenden Dampfes die Liquids A, B und C jeweils ausmachen sollen.

Dabei wird die Leistung der einzelnen Verdampfer 3 vorteilhaft über Pulsweitenmodulierung eingestellt und beispielsweise durch die Messung des Stroms und/oder der Spannung und Einhaltung eines liquidabhängigen Sollwerts geregelt. Einzelne Verdampfer 3 werden vorzugsweise bis maximal 80% ausgelastet, um eine Reserve für beispielsweise einen vom Konsumenten auslösbaren kurzfristigen Leistungsschub (sog. Power Boost) zu erhalten. Zur Auslösung des Power-Boost weist das Gehäuse des Inhalators 27 bzw. des Basisteils 20 vorteilhaft einen von dem Konsumenten betätigbaren Boost-Schalter 30 auf, der elektrisch mit der elektronischen Steuerung 21 verbunden ist.

Figur 8 zeigt eine Verdampfervorrichtung 1 mit zwei Kartuschen 19, die jeweils einen Einkammertank 6 für die Flüssigkeiten A, B aufweisen. Figur 9 zeigt eine Verdampfervorrichtung 1 mit einer Kartusche 19, die einen Zweikammertank 6 für die Flüssigkeiten A, B aufweist. Die Verdampfervorrichtung 1 in Figur 1 entspricht damit der Liquidvarianz der Figuren 8 und 9.

Es ist ersichtlich, dass die Verdampfervorrichtung 1 in Figur 10 die doppelte Menge an Dampf gegenüber denjenigen in Figur 8 oder 9 erzeugen kann. Eine solche erhöhte Dampfmenge ist vorteilhaft über einen Boost-Betätiger 30 erzeugbar, siehe Figur 1. Im geregelten Betrieb kann die maximale Dampfmenge der 80%-Grenze der Verdampfervorrichtung 1 aus Figur 8 entsprechen. So lassen sich sehr flexibel beliebige Kombinationen der Liquids A, B, C erreichen und das Geschmacks- und Wirkungserlebnis ist vom Konsumenten individuell einstellbar. Sollte ein Konsument Dampf mit mehr oder weniger Nikotin bevorzugen oder verschiedene Geschmacksrichtungen kombinieren wollen, so erreicht er dies durch den Einsatz der entsprechenden Verdampfereinheiten 19 und ihrer angebundenen Tanks 6 mit dem präferierten Liquid.

Die in Figur 10 vorgestellte Verdampfervorrichtung 1 wäre sowohl durch die Anordnung in Figur 1 und 3 als auch durch die Anordnung in den Figuren 5 bis 7 realisierbar. Die Verdampfereinheiten 19 bieten durch die standardisierte Schnittstelle 7, 18 den Vorteil einer beliebigen Kombinierbarkeit.

Die elektronische Steuereinrichtung 4 und die Steuerung der Verdampfer 3 sind vorzugsweise spezifisch für jedes im Flüssigkeitsspeicher 6 befindliche Liquid A, B, C programmiert, um eine optimale Aroma- und/oder Wirkstoffgabe zu erzielen.

Um die komplexe Kombinierbarkeit dem Endkunden zur Verfügung zu stellen, müssen die Verdampfer 3 individuell angesteuert werden. Dies wird durch den Einsatz einer Steuereinrichtung 4, beispielsweise eines ASIC, sichergestellt. Über die Schnittstelle 7, 22 werden durch eine Steuerung 21, beispielsweise einen Mikrocontroller, an die Steuereinrichtung 4 Steuerbefehle gesendet, die steuern, welcher Verdampfer 3 mit welchem Tastgrad und mit welcher Frequenz für welchen Zeitraum betrieben wird. Neben den einstellbaren Anteilen der einzelnen Verdampfer 3 bzw. Liquids A, B, ... am Dampf lassen sich die individuellen Verdampfer 3 auch hinsichtlich der Ansteuerfrequenz einstellen und so mit unterschiedlichen Tröpfchengrößen zum Gesamtaerosol beitragen. Dies beeinflusst die Dampfdichte, den Throat Hit und die Wirksamkeit.

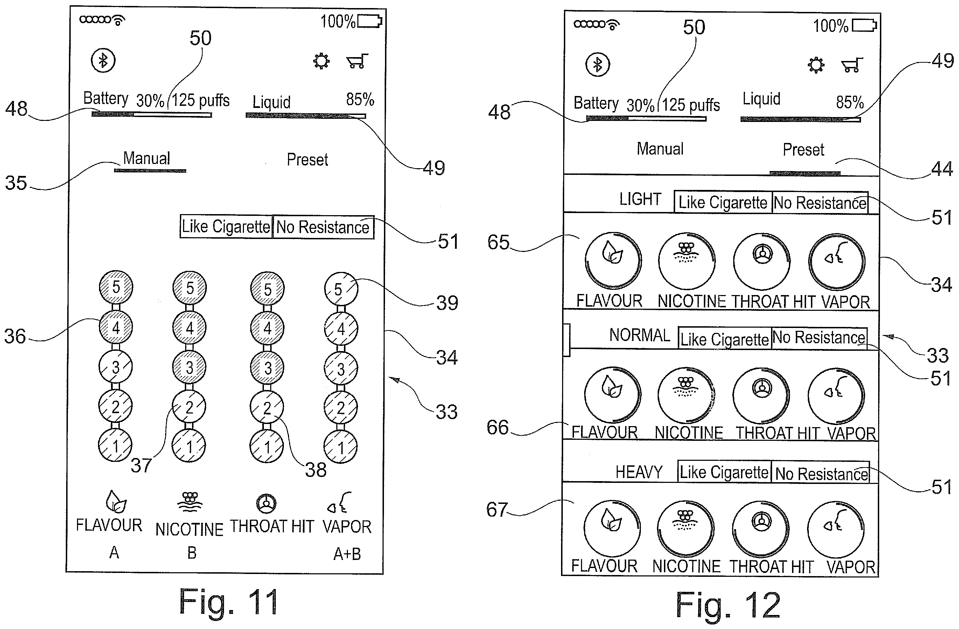

In der Steuerung 21 sind vorzugsweise voreingestellte Verdampfungsprofile gespeichert, welche durch eine Drahtlos-Schnittstelle 32 und mittels einer Applikationssoftware, kurz App, auf einem Smartphone 33 des Konsumenten angepasst werden können. Genauer gesagt weist das Smartphone 33 einen berührungsempfindlichen Bildschirm 34 auf. Die App umfasst eine graphische Benutzerschnittstelle GUI, die eine Mehrzahl von grafischen Bildschirmdarstellungen umfasst, von denen zwei beispielhaft in den Figuren 11 und 12 gezeigt sind.

Die Figuren 11 und 12 beziehen sich auf die individuelle Verdampfung von zwei Flüssigkeiten A, B, beispielsweise mittels einer Verdampfervorrichtung analog zu Figur 2 mit zwei Trägern 2 und jeweils einem Verdampfer 3, vgl. hierzu Figuren 8 und 9. Eines der Flüssigkeitsreservoire 37A enthält beispielsweise ein Liquid mit Flavour, aber ohne Nikotin. Das andere der Flüssigkeitsreservoire 37B enthält Nikotin, aber kein Flavour.

Mit dem entsprechend vorgestellten Inhalator 27 und der App kann der Konsument sein Raucherlebnis weitestgehend unter Vorgabe einer gewünschten Maximaldampfmenge einstellen.

Vorteilhaft umfasst die App die in Figur 11 gezeigte Bildschirmdarstellung, die eine detaillierte und differenzierte Steuerung oder Einstellung des Inhalators 27 ermöglicht und die daher als Expertenmodus oder manueller Modus bezeichnet werden kann. Dieser Modus kann beispielsweise durch Betätigen einer entsprechenden Schaltfläche 35, die in den Figuren 11 und 12 mit "Manual" bezeichnet ist, vom Konsumenten aufgerufen werden. Wenn die in Figur 11 gezeigte manuelle Bildschirmdarstellung angezeigt wird und die App sich im manuellen Modus befindet, kann dies grafisch angezeigt werden, hier beispielsweise durch einen hervorgehobenen Balken im unteren Bereich der Schaltfläche 35.

In dem manuellen Modus kann vorteilhaft jedes im Inhalator 27 enthaltene Liquid A, B separat angesteuert werden. Beispielhaft kann in Figur 11 die Intensität des Flavours aus Liquid A im Dampf/Aerosol zwischen minimal ("soft", Stufe 1) und maximal ("strong", Stufe 5) durch Antippen der entsprechenden Stufe in der Stufenleiste 36 eingestellt werden. In ähnlicher Weise kann die Intensität des Nikotins aus Liquid B im Dampf/Aerosol zwischen minimal ("light", Stufe 1) und maximal ("heavy", Stufe 5) durch Antippen der entsprechenden Stufe in der Stufenleiste 37 eingestellt werden.

Demnach kann der zwischen einem 100%-igen Nikotinerlebnis (Regler 36 auf Maximum), einem 100%-igen Geschmackserlebnis (Regler 37 auf Maximum) oder einer Mischung aus beidem wählen.

Eine weitere Einstellbarkeit im manuellen Modus kann den sogenannten Throat Hit betreffen, also eine kurzfristige Wirkung in der Kehle des Konsumenten. Der Throat Hit korreliert dabei mit dem Nikotin und der jeweiligen Gesamtmenge an Propylenglycol im Dampf. Der Throat Hit, also die Intensität von Nikotin aus Liquid B und Propylenglycol im Dampf/Aerosol kann in Figur 11 gezielt zwischen minimal ("soft", Stufe 1) und maximal ("strong", Stufe 5) durch Antippen der entsprechenden Stufe in der Stufenleiste 38 eingestellt werden.

Vorteilhaft kann des Weiteren im manuellen Modus die gesamte Dampfmenge zwischen minimal ("little", Stufe 1) und maximal ("plenty", Stufe 5) durch Antippen der entsprechenden Stufe in der Stufenleiste 39 eingestellt werden. Die maximale Dampfmenge entspricht dabei dem gleichzeitigen Betrieb sämtlicher (hier beider) Verdampfer 3 der Verdampfungseinrichtung 1. Die eingestellte Dampfmenge ergibt sich somit vorteilhaft aus Summe der Verdampfungsintensitäten der einzelnen Liquids A und B.

Selbstverständlich können anstelle der Stufenleisten 35-38 alternativ Schiebeleisten oder beliebige andere geeignete mehrstufige Betätigungselemente vorgesehen sein.

Die in dem manuellen Modus einstellbaren Verdampfungsparameter sind voneinander abhängig. Wenn beispielsweise mit dem Einstellelement 37 die Verdampfung des Liquids B, und somit der Nikotingehalt in dem erzeugten Dampf/Aerosol, auf Null eingestellt wird, kann die gesamte Dampfmenge im vorliegenden Beispiel maximal halb so groß sein wie bei maximal eingestelltem Nikotingehalt, weil nur das Liquid A mit der Hälfte aller Verdampfer 3 verdampft wird. Ähnliches gilt, wenn eines der beiden Liquids A, B erschöpft ist.

Die App berücksichtigt sämtliche Abhängigkeiten der Verdampfungsparameter untereinander und passt die Bildschirmdarstellungen entsprechend an. Beispielsweise würde in dem oben genannten Beispiel beim Herunterfahren der Nikotinmenge mittels des Einstellelements 37 gleichzeitig und automatisch auch das Einstellelement 39 für die Dampfmenge auf maximal den halben Wert heruntergefahren werden.

Für den Preset-Modus gemäß Figur 12 gilt entsprechendes. Wenn beispielsweise das Liquid B erschöpft ist, können Profile, die einen bestimmten Mindest-Nikotingehalt aufweisen, beispielsweise nicht mehr auswählbar sein.

Vorteilhaft umfasst die App weiterhin die in Figur 11 gezeigte Bildschirmdarstellung, die eine vereinfachte Steuerung oder Einstellung des Inhalators 27 ermöglicht und die daher als vereinfachter Modus oder Preset-Modus bezeichnet werden kann. Dieser Modus kann beispielsweise durch Betätigen einer entsprechenden Schaltfläche 44 aufgerufen werden, die in den Figuren 11 und 12 mit "Preset" bezeichnet ist. Wenn die in Figur 12 gezeigte manuelle Bildschirmdarstellung angezeigt wird und die App sich im Preset-Modus befindet, kann dies grafisch angezeigt werden, hier beispielsweise durch einen hervorgehobenen Balken im unteren Bereich der Schaltfläche 44.

In dem vereinfachten oder Preset-Modus kann vorteilhaft eine Mehrzahl, beispielsweise drei, von unterschiedlichen, vordefinierten Verdampfungsprofilen vom Konsumenten ausgewählt werden. Beispielsweise sind in Figur 12 drei unterschiedliche Profile leicht ("Light"), normal und stark ("Heavy") gezeigt. Jedes Profil ist mittels einer entsprechenden Schaltfläche 65, 66, 67 vom Konsumenten auswählbar.

In Figur 12 ist beispielsweise das Profil "normal" ausgewählt, was durch eine Hervorhebung, hier einem hervorgehobenen Balken links neben dem Schriftzug "Normal" angezeigt wird. Jedes Profil umfasst ein voreingestelltes Niveau für die in Figur 11 gezeigten individuellen Parameter. Beispielsweise ist für das Profil "normal" in Figur 12 voreingestellt: Flavour A Stufe 3; Nikotin B Stufe 3; Throat Hit Stufe 3; Dampfmenge Stufe 4. Für das Profil "Heavy" ist in Figur 12 beispielsweise voreingestellt: Flavour A Stufe 2; Nikotin B Stufe 4; Throat Hit Stufe 4; Dampfmenge Stufe 2.

Aufgrund der auf das Wesentliche beschränkten Wahlmöglichkeiten kann der Konsument unkompliziert und ohne viel Nachdenken ein gewünschtes Raucherlebnis einstellen.

Zusätzlich oder alternativ kann der Inhalator 27 einen am Gehäuse angeordneten Schalter 54, beispielsweise eine Schiebeschalter, aufweisen, der ebenfalls eine Auswahl von Verdampfungsprofilen ermöglicht.

Vorzugsweise umfasst die App eine Anzeige 48 des Ladezustands des Energiespeichers 46 in dem Inhalator 27, beispielsweise in Prozent des maximalen Ladezustands, und/oder als Anzeige der verbleibenden Züge durch den Konsumenten (sog. Puffs).

Vorzugsweise umfasst die App eine Anzeige 49 der restlichen Liquidmenge in dem oder den Reservoirs 37A, 37B, ... des Inhalators 27, beispielsweise in Prozent der maximalen Liquidmenge. Die Anzeige 49 betrifft hier die gesamte Flüssigkeitsmenge in sämtlichen Reservoirs 37A, 37B, .... Es kann jedoch auch die individuelle Flüssigkeits-Restmenge in jedem einzelnen Reservoir 37A, 37B, ... angezeigt werden.

Die App kann des Weiteren vorteilhaft eine Anzeige 50 der verbleibenden Züge durch den Konsumenten (sog. Puffs) aufweisen. Die Anzahl der verbleibenden Puffs kann abhängig von den gewählten Einstellungen, dem Rauchverhalten, beispielsweise der durchschnittlichen individuellen Zuglängen, und dem Energiebedarf berechnet werden.

Schließlich kann die App ein Schaltelement 51 zum Einstellen des Zugwiderstands des Inhalators 27, mittels eines den Zugwiderstand ändernden Elements 52 in dem Inhalator 27 (siehe Figur 1), aufweisen. Im Beispiel der Figuren 11 und 12 können mit dem Schaltelement 51 zwei unterschiedliche Zugwiderstände eingestellt werden, nämlich ein Zugwiderstand, der demjenigen einer klassischen Zigarette entspricht (linke Schalterposition "Like Cigarette"), und einen möglichst geringen Zugwiderstand (rechte Schalterposition "No Resistance"). Die Einstellung von drei oder mehr unterschiedlichen Zugwiderständen ist möglich.

Die Anzeigen 48, 49, 50 und/oder 51 können vorteilhaft auf sämtlichen Bildschirmdarstellungen der App dargestellt werden.

Zusätzlich oder alternativ kann der Inhalator 27 einen am Gehäuse angeordneten Schalter 55, beispielsweise eine Schiebeschalter, aufweisen, der ebenfalls eine Auswahl unterschiedlicher Zugwiderstände des Inhalators 27 durch Betätigung des den Zugwiderstand ändernden Elements 52 ermöglicht. Element 52 und Schalter 55 können auch ein und dasselbe Teil sein.

Die App kann eine nicht gezeigte Schaltfläche zur Auslösung eines Boost aufweisen, zusätzlich oder alternativ zu dem beispielsweise mechanischen Boost-Schalter 30 an dem Inhalator 27.

Die zuvor beschrieben Kombination aus Verdampfern 3, Trägern 2 und Reservoirs 37A, 37B, ... erlaubt eine große Vielfalt an Einstellmöglichkeiten und somit einen hohen Mehrwert für den Konsumenten aufgrund einer hochgradigen Individualisierbarkeit des Raucherlebnisses in Dampfmenge und Geschmack.

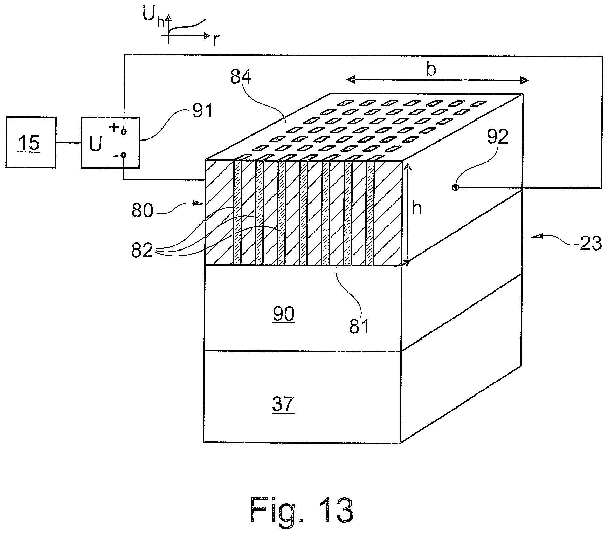

Eine vorteilhafte Ausführungsform eines erfindungsgemäßen Verdampfers 3 ist in Fig. 13 gezeigt. Der Verdampfer 3 ist hier durch einen blockförmigen, vorteilhaft monolithischen Heizkörper 80 beziehungsweise einen Volumenheizkörper aus einem elektrisch leitenden Material, nämlich Silizium, und vorzugsweise zusätzlich dotierte Keramik, Metall-Keramik, Filter-Keramik, Halbleiter, insbesondere Germanium, Graphit, Halbmetall und/oder Metall gebildet. Es ist nicht erforderlich, dass der gesamte Heizkörper 80 aus einem elektrisch leitenden Material besteht. Es kann beispielsweise ausreichen, dass die Oberfläche des Heizkörpers 80 elektrisch leitend, beispielsweise metallisch, beschichtet ist. In diesem Fall muss nicht die gesamte Oberfläche beschichtet sein, beispielsweise können Leiterbahnen auf einem nichtleitenden Grundkörper vorgesehen sein. Volumenheizer ermöglichen eine kompaktere Bauweise als Wendelheizer, sonstige Drahtheizer und/oder Filamentheizer.

Der Heizkörper 80 ist mit einer Mehrzahl von Mikrokanälen 82 versehen, die eine Einlassseite 81 des Heizkörpers 80 mit einer Auslassseite 84 flüssigkeitsleitend verbinden. Die Einlassseite 81 ist beispielsweise über eine Dochtstruktur 90 flüssigkeitsleitend mit dem Flüssigkeitsspeicher 37 verbunden. Die Dochtstruktur 90 dient zur passiven Förderung von Flüssigkeit aus dem Flüssigkeitsspeicher 37 zu dem Heizkörper 80 mittels Kapillarkräften. Die Dochtstruktur 90 im Kontaktbereich 81 zu dem Heizkörper 80 dient dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Heizkörper 80 in die Dochtstruktur 90 und/oder in den Flüssigkeitsspeicher 37 zu verhindern. Anstelle der Dochtstruktur 90 können andere passive und/oder aktive Fördereinrichtungen zum Fördern von Flüssigkeit aus dem Flüssigkeitsspeicher 37 zu dem Heizkörper 80 vorgesehen sein.

Der mittlere Durchmesser der Mikrokanäle 82 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 81 in einen Mikrokanal 82 eindringende Flüssigkeit durch den Mikrokanal 82 nach oben steigt, bis der Mikrokanal 82 mit Flüssigkeit gefüllt ist. Das Volumenverhältnis von Mikrokanälen 82 zu Heizkörper 80, das als Porosität des Heizkörpers 80 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Mikrokanälen 82 versehenen Flächen des Heizkörpers 80 liegen beispielsweise im Bereich zwischen 0,5 mm und 3 mm. Die Abmessungen der mit Mikrokanälen 82 versehenen Flächen des Heizkörpers 80 können beispielsweise betragen:
0,95 mm x 1,75 mm; 1,9 mm x 1,75 mm oder 1,9 mm x 0,75 mm. Die Kantenlängen des Heizkörpers 80 können beispielsweise im Bereich zwischen 0,5 mm und 5 mm liegen, vorzugsweise im Bereich zwischen 0,75 mm und 4 mm, weiter vorzugsweise im Bereich zwischen 1 mm und 3 mm liegen. Die Fläche des Heizkörpers 80 (chip size) kann beispielsweise 1 mm x 3 mm oder 2 mm x 3 mm betragen.

Die Breite b des Heizkörpers 80 liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörpers 80 liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 82 liegt vorzugsweise im Bereich zwischen vier und 1000. Auf diese Weise lässt sich der Wärmeeintrag von dem Träger in die Mikrokanäle 82 optimieren und eine gesicherte hohe Verdampfungsleistung sowie eine ausreichend große Dampfaustrittsfläche realisieren.

Die Mikrokanäle 82 sind vorteilhaft in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 82 mit gesichert hoher Verdampfungsleistung realisieren.

Der Querschnitt der Mikrokanäle 82 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 82 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von dem Heizkörper 80 in die Mikrokanäle 82 realisieren.

Der Abstand zweier Mikrokanäle 82 beträgt vorzugsweise mindestens das 1,3-fache des lichten Durchmessers eines Mikrokanals 82, wobei der Abstand auf die Mittelachsen der beiden Mikrokanäle 82 bezogen ist. Der Abstand kann bevorzugt das 1,5- bis 5-fache, weiter bevorzugt das 2- bis 4-fache des lichten Durchmessers eines Mikrokanals 82 betragen. Auf diese Weise lässt sich ein optimaler Wärmeeintrag von dem Träger in die Mikrokanäle und eine ausreichend stabile Anordnung und Wandstärke der Mikrokanäle realisieren.

Die Verdampfereinheit 20 weist eine vorzugsweise von der Steuervorrichtung 56 steuerbare Heizspannungsquelle 91 auf, die über Elektroden 92 an gegenüberliegenden Seiten des Heizkörpers 80 mit diesem verbunden ist, so dass eine von der Heizspannungsquelle 91 erzeugte elektrische Spannung Uh zu einem Stromfluss durch den Heizkörper 80 führt. Aufgrund des Ohm'schen Widerstands des elektrisch leitenden Heizkörpers 80 führt der Stromfluss zu einer Erhitzung des Heizkörpers 80 und daher zu einer Verdampfung von in den Mikrokanälen 82 enthaltener Flüssigkeit. Der Heizkörper 80 wirkt somit als Verdampfer 3.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Der Heizkörper 80 kann vorteilhaft aus Teilstücken eines Wafers mit Dünnfilmschichttechnologie hergestellt werden, welcher eine gebräuchliche Schichtdicke aufweist.

Die Verdampfer 3 können so eingestellt sein, dass eine vorteilhafte Flüssigkeitsmenge im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise können die Verdampfer 3 hinsichtlich der Flüssigkeitsmenge pro Zug einstellbar sein.

Die Verdampfer 3 können vorteilhaft so eingestellt sein, dass überwiegend Flüssigkeitstropfen mit einem Durchmesser im Bereich zwischen 0,05 µm und 5 µm, bevorzugt zwischen 0,1 µm und 3 µm entstehen. Tröpfchengrößen im Bereich zwischen 0,05 und 5 MMAD (mass median aerodynamic diameter, massen-medianer aerodynamischer Durchmesser), vorzugsweise zwischen 0,1 und 3 MMAD, weiter vorzugsweise zwischen 0,5 und 2 MMAD, noch weiter vorzugsweise zwischen 0,7 und 1,5 MMAD, beispielsweise um ca. 1 MMAD können optimal sein. MMAD entspricht einer EU-Norm und wird in µm spezifiziert.

Die von der Heizspannungsquelle 91 erzeugte Ansteuerfrequenz des Heizkörpers 80 liegt vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Im Folgenden wird der Ablauf des Verdampfungsvorgangs erläutert.

In einem Ausgangszustand ist die Spannungsquelle 91 für den Heizvorgang ausgeschaltet.

Zum Verdampfen von Flüssigkeit wird die Spannungsquelle 91 für den Heizkörper 80 aktiviert. Die Spannung Uh wird dabei so eingestellt, dass die Verdampfungstemperatur in dem Heizkörper 80 und somit in den Mikrokanälen 82 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der Mikrokanäle 82 entspricht oder damit in Zusammenhang steht, wird die Heizspannungsquelle 91 deaktiviert. Da die Liquideigenschaften und -menge vorteilhaft exakt bekannt sind, kann dieser Zeitpunkt sehr genau gesteuert werden. Die Energieaufnahme der Verdampfer 3 lässt sich daher gegenüber bekannten Vorrichtungen reduzieren, da die benötigte Verdampfungsenergie dosierter und damit exakter eingebracht werden kann.

Nach Abschluss des Heizvorgangs sind die Mikrokanäle 82 überwiegend oder vollständig entleert. Die Heizspannung 91 wird dann so lange ausgeschaltet gehalten, bis mittels Nachförderung von Flüssigkeit durch die Dochtstruktur 90 die Mikrokanäle 82 wieder aufgefüllt sind. Sobald dies der Fall ist, kann der nächste Heizzyklus durch Einschalten der Heizspannung 91 begonnen werden.

Der Heizkörper 80 ist vorzugsweise auf der Grundlage von MEMS-Technologie, insbesondere aus Silizium, gefertigt und daher vorteilhaft ein Mikro-Elektro-Mechanisches System.

## Patentansprüche

1. Inhalator (27), insbesondere elektronisches Zigarettenprodukt, umfassend mindestens eine Verdampfervorrichtung (1) mit mindestens einem elektrischen Verdampfer (3) zum Verdampfen von dem Verdampfer (3) zugeführter Flüssigkeit und eine elektronische Steuervorrichtung (56) zum Steuern und/oder Regeln des Verdampfers (3), wobei der Verdampfervorrichtung (1) eine Mehrzahl von Flüssigkeiten zugeordnet oder zuordnungsbar sind, derart, dass die Zusammensetzung und/oder die Dampferzeugungsrate des von dem mindestens einen Verdampfer erzeugten Dampfs/Aerosols gezielt einstellbar und/oder veränderbar ist, indem die Steuervorrichtung (56) zum individuellen Ansteuern des mindestens einen oder des individuell jeweiligen Verdampfers (3) und/oder zum gruppenweisen Ansteuern von Verdampfern (3) eingerichtet ist, **dadurch gekennzeichnet, dass**
- die Verdampfervorrichtung (1) mindestens einen Träger (2) und mindestens einen mit dem Träger (2) zu verbindenden oder verbundenen Flüssigkeitsspeicher (6) umfasst, wobei auf jedem Träger (2) mindestens ein Verdampfer (3) angeordnet ist, wobei
- jeder Träger (2) über eine standardisierte Schnittstelle (7) zur elektrischen Kontaktierung des oder der Verdampfer (3) verfügt, wobei
- der Verdampfer (3) durch einen blockförmigen Heizkörper (80) aus einem elektrisch leitenden Material, nämlich Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden.

2. Inhalator nach Anspruch 1, **dadurch gekennzeichnet, dass** die Verdampfervorrichtung (1) eine Mehrzahl von Flüssigkeitszuführungen (16) zum Zuführen der Mehrzahl von Flüssigkeiten A, B, ... zu dem mindestens einen Verdampfer (3) aufweist.

3. Inhalator nach Anspruch 1 oder 2, **dadurch gekennzeichnet, dass** in der elektronischen Steuervorrichtung (56) eine Mehrzahl von an die mehreren Flüssigkeiten A, B, C angepasste Parametersätze gespeichert sind.

4. Inhalator nach einem der vorangehenden Ansprüche, **dadurch gekennzeichnet, dass** die Verdampfervorrichtung (1) eine Mehrzahl von Verdampfern (3) aufweist.

5. Inhalator nach Anspruch 4, **dadurch gekennzeichnet, dass** jeder Flüssigkeit A, B, ... mindestens ein eigener Verdampfer (3) zugeordnet ist.

6. Inhalator nach einem der vorangehenden Ansprüche, **dadurch gekennzeichnet, dass** der Inhalator (27) eine Nutzerschnittstelle (32) aufweist, über welche ein Nutzer die Zusammensetzung des von dem mindestens einen Verdampfer (3) erzeugten Dampfs / Aerosols beeinflussen kann.

7. Inhalator nach einem der vorangehenden Ansprüche, **dadurch gekennzeichnet, dass** die Nutzerschnittstelle (32) eine Drahtlosschnittstelle zur Kommunikation mit einem mobilen Kommunikationsendgerät (33) des Nutzers, insbesondere einem Smartphone, ist.

8. Inhalator nach einem der vorangehenden Ansprüche, **dadurch gekennzeichnet, dass** die Flüssigkeiten A, B, ... eine oder mehrere Komponenten aus der Gruppe 1,2-Propylenglycol, Glycerin, Wasser, mindestens ein Wirkstoff, insbesondere Nikotin, und/oder mindestens ein Aromastoff (Flavour) enthalten.

9. Inhalator nach einem der vorangehenden Ansprüche, **dadurch gekennzeichnet, dass** mindestens eine der Flüssigkeiten A, B, ... mehr als 50% Glycerin enthält.

10. Inhalator nach einem der vorangehenden Ansprüche, **dadurch gekennzeichnet, dass** mindestens eine der Flüssigkeiten A, B, ... mindestens 1% Nikotin und mindestens eine der Flüssigkeiten A, B, ... kein Nikotin enthält.

11. Inhalator nach einem der Ansprüche 1 bis 9, **dadurch gekennzeichnet, dass** jede der dem mindestens einem Verdampfer (3) zugeführten Flüssigkeiten A, B, ... frei von Nikotin ist.

12. Inhalator nach einem der vorangehenden Ansprüche, **dadurch gekennzeichnet, dass** mindestens eine der Flüssigkeiten A, B, ... ein Flavour und mindestens eine der Flüssigkeiten A, B, ... kein Flavour, oder ein anderes Flavour, enthält.

13. Inhalator nach einem der vorangehenden Ansprüche, **dadurch gekennzeichnet, dass** der Inhalator (27) eine Mehrzahl von Reservoirs (37A, 37B, ...) zum Speichern der Mehrzahl von Flüssigkeiten A, B, ... aufweist.

14. Inhalator nach Anspruch 13, **dadurch gekennzeichnet, dass** mindestens eine der Flüssigkeiten A, B, ... mehrheitlich eine oder mehrere Komponenten aus der Gruppe 1,2-Propylenglycol enthält und eine andere der Flüssigkeiten A, B, ... mehrheitlich Glycerin enthält.

15. Inhalator nach einem der vorangehenden Ansprüche, **dadurch gekennzeichnet, dass** der Inhalator (27) wenigstens eine Kapsel zum Speichern eines aroma- und/oder wirkstoffhaltigen, insbesondere nikotinhaltigen, Feststoffes aufweist, wobei die Kapsel von dem von dem mindestens einen Verdampfer erzeugten Dampf/Aerosol durchströmbar ist.

## Claims

1. Inhaler (27), in particular electronic cigarette product, comprising at least one vaporizer device (1) with at least one electric vaporizer (3) for vaporizing liquid supplied to the vaporizer (3) and an electronic control device (56) for controlling and/or regulating the vaporizer (3), wherein a plurality of liquids are or can be assigned to the vaporizer device (1), in such a way that the composition and/or the vapour generation rate of the vapour/aerosol generated by the at least one vaporizer can be set and/or changed in a targeted manner, in that the control device (56) is adapted to control individually the at least one or the individually respective vaporizer (3) and/or to control vaporizers (3) in groups, **characterized in that**
- the vaporizer device (1) comprises at least one carrier (2) and at least one liquid reservoir (6) to be connected to or connected to the carrier (2), wherein at least one vaporizer (3) is arranged on each carrier (2), wherein
- each carrier (2) has a standardized interface (7) for electrical contacting of the vaporizer or vaporizers (3), wherein
- the vaporizer (3) is formed by a block-shaped heating body (80) made of an electrically conductive material, namely silicon, wherein the heating body (80) is provided with a plurality of microchannels (82) which connect an inlet side (81) of the heating body (80) to an outlet side (84) in a liquid-conducting manner.

2. Inhaler according to claim 1, **characterized in that** the vaporizer device (1) comprises a plurality of liquid feeds (16) for feeding the plurality of liquids A, B, ... to the at least one vaporizer (3).

3. Inhaler according to claim 1 or 2, **characterized in that** a plurality of parameter sets adapted to the plurality of liquids A, B, C are stored in the electronic control device (56).

4. Inhaler according to any one of the preceding claims, **characterized in that** the vaporizer device (1) comprises a plurality of vaporizers (3).

5. Inhaler according to claim 4, **characterized in that** each liquid A, B, ... has at least one dedicated vaporizer (3) assigned to it.

6. Inhaler according to any one of the preceding claims, **characterized in that** the inhaler (27) comprises a user interface (32) via which a user can affect the composition of the vapor/aerosol generated by the at least one vaporizer (3).

7. Inhaler according to any one of the preceding claims, **characterized in that** the user interface (32) is a wireless interface for communication with a mobile communication device (33) of the user, in particular a smartphone.

8. Inhaler according to any one of the preceding claims, **characterized in that** the liquids A, B, ... contain one or more components from the group 1,2-propylene glycol, glycerol, water, at least one active substance, in particular nicotine, and/or at least one flavoring substance (flavor).

9. Inhaler according to any one of the preceding claims, **characterized in that** at least one of the liquids A, B, ... contains more than 50% glycerol.

10. Inhaler according to any one of the preceding claims, **characterized in that** at least one of the liquids A, B, ... contains at least 1% nicotine and at least one of the liquids A, B, ... does not contain nicotine.

11. Inhaler according to any one of claims 1 to 9, **characterized in that** each of the liquids A, B, ... supplied to the at least one vaporizer (3) is free of nicotine.

12. Inhaler according to any one of the preceding claims, **characterized in that** at least one of the liquids A, B, ... contains a flavor and at least one of the liquids A, B, ... contains no flavor, or another flavor.

13. Inhaler according to any one of the preceding claims, **characterized in that** the inhaler (27) comprises for storing the plurality of liquids A, B, ... a plurality of reservoirs (37A, 37B, ...).

14. Inhaler according to claim 13, **characterized in that** at least one of the liquids A, B, ... comprises a majority of one or more components from the group 1,2-propylene glycol and another of the liquids A, B, ... comprises a majority of glycerol.

15. Inhaler according to any one of the preceding claims, **characterized in that** the inhaler (27) comprises at least one capsule for storing an aroma- and/or active substance-containing, in particular nicotine-containing, solid, wherein the capsule can be flowed through by the vapor/aerosol generated by the at least one vaporizer.

## Revendications

1. Inhalateur (27), notamment produit cigarette électronique, comprenant au moins un dispositif d'atomiseur (1), avec au moins un atomiseur électrique (3) pour vaporiser du liquide alimenté dans l'atomiseur (3), et un dispositif de commande électronique (56) pour commander et/ou réguler l'atomiseur (3), une pluralité de liquides étant ou pouvant être associés au dispositif d'atomiseur (1), de telle manière que la composition et/ou le taux de génération de vapeur de la vapeur / de l'aérosol généré(e) par l'au moins un atomiseur peuvent être réglés et/ou modifiés de manière ciblée, du fait que le dispositif de commande (56) est configuré pour la commande individuelle de l'au moins un atomiseur (3) ou de l'atomiseur (3) individuellement respectif et/ou pour la commande groupée d'atomiseurs (3), **caractérisé en ce que**
- le dispositif d'atomiseur (1) comprend au moins un support (2) et au moins un réservoir de liquide (6) à connecter ou connecté avec le support (2), au moins un atomiseur (3) étant disposé sur chaque support (2), dans lequel
- chaque support (2) dispose d'une interface (7) normalisée pour la mise en contact électrique du ou des atomiseurs (3), dans lequel
- l'atomiseur (3) est formé par un corps chauffant (80) en forme de bloc constitué d'un matériau électriquement conducteur, à savoir du silicium, le corps chauffant (80) étant pourvu d'une pluralité de microcanaux (82), qui relient un côté entrée (81) du corps chauffant (80) à un côté sortie (84) de manière à permettre un passage de liquide.

2. Inhalateur selon la revendication 1, **caractérisé en ce que** le dispositif d'atomiseur (1) comporte une pluralité d'alimentations de liquide (16) pour alimenter la pluralité de liquides A, B, ... dans l'au moins un atomiseur (3).

3. Inhalateur selon la revendication 1 ou 2, **caractérisé en ce qu'**une pluralité de jeux de paramètres adaptés à la pluralité de liquides A, B, C sont enregistrés dans le dispositif de commande électronique (56).

4. Inhalateur selon l'une des revendications précédentes, **caractérisé en ce que** le dispositif d'atomiseur (1) comporte une pluralité d'atomiseurs (3).

5. Inhalateur selon la revendication 4, **caractérisé en ce qu'**au moins un atomiseur (3) propre est associé à chaque liquide A, B, ...

6. Inhalateur selon l'une des revendications précédentes, **caractérisé en ce que** l'inhalateur (27) comporte une interface utilisateur (32), par le biais de laquelle un utilisateur peut influencer la composition de la vapeur / de l'aérosol généré(e) par l'au moins un atomiseur (3).

7. Inhalateur selon l'une des revendications précédentes, **caractérisé en ce que** l'interface utilisateur (32) est une interface sans fil pour la communication avec un terminal de communication mobile (33) de l'utilisateur, en particulier un mobile multifonction.

8. Inhalateur selon l'une des revendications précédentes, **caractérisé en ce que** les liquides A, B, ... contiennent un ou plusieurs composants du groupe propane-1,2-diol, glycérine, eau, au moins une substance active, en particulier de la nicotine, et/ou au moins une substance aromatisante (arôme).

9. Inhalateur selon l'une des revendications précédentes, **caractérisé en ce qu'**au moins un des liquides A, B, ... contient plus de 50 % de glycérine.

10. Inhalateur selon l'une des revendications précédentes, **caractérisé en ce qu'**au moins un des liquides A, B, ... contient au moins 1 % de nicotine et au moins un des liquides A, B, ... ne contient pas de nicotine.

11. Inhalateur selon l'une des revendications 1 à 9, **caractérisé en ce que** chacun des liquides A, B, ... alimentés dans l'au moins un atomiseur (3) est exempt de nicotine.

12. Inhalateur selon l'une des revendications précédentes, **caractérisé en ce qu'**au moins un des liquides A, B, ... contient un arôme et au moins un des liquides A, B, ... ne contient pas d'arôme ou contient un autre arôme.

13. Inhalateur selon l'une des revendications précédentes, **caractérisé en ce que** l'inhalateur (27) comporte une pluralité de réservoirs (37A, 37B, ...) pour stocker la pluralité de liquides A, B, ...

14. Inhalateur selon la revendication 13, **caractérisé en ce qu'**au moins un des liquides A, B, ... contient majoritairement un ou plusieurs composants du groupe propane-1,2-diol et un autre des liquides A, B, ... contient majoritairement de la glycérine.

15. Inhalateur selon l'une des revendications précédentes, **caractérisé en ce que** l'inhalateur (27) comporte au moins une capsule pour stocker une substance solide contenant un arôme et/ou une substance active, en particulier contenant de la nicotine, la capsule pouvant être traversée par la vapeur / l'aérosol généré(e) par l'au moins un atomiseur.
